# FASCICULE DE BREVET EUROPEEN

(11) **EP 1 175 227 B1**
(45) Date de publication et mention de la délivrance du brevet: **11.04.2007**
(21) Numéro de dépôt: 00925425.1
(22) Date de dépôt: 10.05.2000
(51) Int. Cl.: A61K 39/395, C07K 16/30, C07K 16/42, C07K 14/47, C12N 5/18, G01N 33/577, G01N 33/574, A61K 47/48

(54) **ANTICORPS MONOCLONAL DIRIGE CONTRE LES CELLULES DE CARCINOME RENAL HUMAIN**
MONOKLONALER ANTIKÖRPER GEGEN MENSCHLISCHE NIERENKARZINOMZELLEN
MONOCLONAL ANTIBODY DIRECTED AGAINST CELLS OF HUMAN RENAL CELL CARCINOMA

(30) Priorité: 10.05.1999 FR 9905942
(43) Date de publication de la demande: 30.01.2002
(73) Titulaire: CENTRE NATIONAL DE LA RECHERCHE SCIENTIFIQUE, 75016 Paris Cédex (FR)
(72) Inventeur: HIRSCH, François, F-94110 Arcueil (FR); ANGEVIN, Eric, F- 45700 Villevoques (FR)
(74) Mandataire: Warcoin, Jacques
(86) Numéro de dépôt international: PCT/FR2000/001258
(87) Numéro de publication internationale: WO 2000/067792

(56) Documents cités:
- EP-A- 0 160 250
- WO-A-88/08854

## Description

La présente invention concerne un nouvel hybridome produisant un anticorps monoclonal dirigé spécifiquement contre un antigène de carcinome de cellules rénales humaines (RCC); l'invention concerne également l'utilisation de cet anticorps monoclonal notamment pour le diagnostic, l'imagerie et le traitement des RCC.

Les RCC sont des cancers particulièrement difficiles à diagnostiquer dans les formes disséminées (Oberling *et al.* (1960) Nature 186, 402-403; Holthafer *et al*. (1983) Lab. Invest. 49, 319-326) qui représentent chaque année environ 5% des nouveaux cas de cancers. En dehors de la chirurgie de première intention, il n'y a, à l'heure actuelle, aucun traitement efficace en cas de récidive.

Un anticorps monoclonal (mAb) spécifique d'antigène présent de manière prédominante uniquement sur les cellules de carcinome rénal humain serait extrêmement utile pour le diagnostic, la localisation et le traitement de cette maladie. Un nombre limité de mAb dirigés contre les RCC humains ont été rapporté dans la littérature (Oosterwijk E *et al.* (1986) Int. J. Cancer 38: 489-494; Kinouchi T. *et al.* (1995) J. Urol. 154: 288-292; Kaufmann O. *et al.* (1997) Histopathology 31:31-37) ou ont fait l'objet de brevets ou de demandes de brevet (EP 119 528, EP 160 250, EP 210 970, WO 88/08854, WO 90/14595).

Parmi les anticorps actuellement disponibles, l'anticorps G250, par exemple, n'a pas les qualités requises pour le diagnostic, l'imagerie et/ou la thérapie. Ainsi, l'anticorps G250 (WO 88/08854, inventeurs Oosterwijk E. et Warnaar S.O.) le plus étudié et le plus utilisé des mAb dirigés contre les RCC puisqu'utilisé dans deux études cliniques d'immunoradiolocalisation de tumeurs primitives ou métastasées (Oosterwijk E. *et al.* (1993) J. Clin. Oncol. 11: 738-750; Steffens M.G. *et al.* (1997) J. Clin. Oncol. 15: 1529-1537), ne reconnaît que 70 à 80% des tumeurs. Cependant à ce jour, aucun effet thérapeutique n'a été obtenu avec l'anticorps G250. Une autre limite de l'exploitation de cet anticorps réside dans le fait que les tumeurs humaines exprimant l'antigène G250 ne peuvent se développer que très difficilement chez la souris, rendant difficile la réalisation d'un bon modèle animal de ciblage *in vivo,* via le mAb G250.

Ces résultats soulignent donc la nécessité d'obtenir d'autres anticorps monoclonaux spécifiques d'un antigène différent et reconnaissant de manière hautement spécifique les RCC sans présenter de réactivité substantielle avec les cellules normales.

Les inventeurs ont employé la technique des hybridomes développée à l'origine par Köhler et Milstein (Nature (1975) 256: 495-497) pour obtenir un nouvel hybridome produisant un nouvel anticorps monoclonal hautement spécifique des carcinomes rénaux humains et reconnaissant un antigène spécifique différent de l'antigène G250. La présente invention a donc pour objet un hybridome désigné 5C5tel que déposé à la CNCM sous le numéro I-2184, formé par la fusion de cellules d'une lignée de myélome murin Balb/C Sp2O et de cellules de ganglions inguinaux et abdominaux de souris Balb/C ayant été préalablement immunisée avec la lignée de carcinome rénal humain BIZ.

La présente invention concerne également un anticorps monoclonal spécifique de la plupart des cellules de carcinomes rénaux humains appelé 5C5, ne se liant pas à l'antigène G250 et susceptible d'être obtenu par l'hybridome de l'invention. L'anticorps monoclonal ainsi produit a été isolé puis caractérisé. La sous-classe et l'isotype de l'anticorps monoclonal ont été déterminé par la technique d'immunoprécipitation de Ouchterlony qui utilise des anticorps de lapin dirigés contre des sous-classes d'immunoglobulines de souris.

La caractérisation de l'anticorps monoclonal 5C5 de la présente invention a été réalisée sur des lignées cellulaires de RCC ainsi que sur des fragments tissulaires de patients atteints de RCC. L'analyse de la présence de l'antigène de cellules de carcinome rénal spécifiquement reconnu par l'anticorps de la présente invention peut être réalisée par des techniques d'immunohistochimie utilisant par exemple des colorations à la peroxydase ou par des techniques d'immunofluorescence. Selon un protocole standard, les lames sur lesquelles sont posées les sections gelées, obtenues au cryostat, des échantillons non fixés de biopsie sont séchées à l'air puis incubées avec l'anticorps monoclonal 5C5 dans un chambre humide à température ambiante. Les lames sont ensuite recouvertes d'une préparation d'anticorps dirigée contre l'anticorps ou le fragment d'anticorps de la présente invention. Par exemple, si l'anticorps monoclonal 5C5 est utilisé, le second anticorps peut être un anticorps anti-souris. Le second anticorps peut être marqué avec un composé fluorescent (fluorescéine, Texas-red, DAPI). La nature du marquage de l'échantillon ainsi que son intensité sont ensuite déterminées à l'aide d'un microscope à fluorescence. Dans des expériences d'immunofluorescence, l'anticorps selon l'invention se caractérise en ce que ledit anticorps ou fragment d'anticorps se lie de manière spécifique à un antigène commun aux cellules de lignées de carcinome rénal humain BIZ, HIEG, QUIE, BENA, PIUZ, GUIL, GUER, ROB, DURI, JOUS, GUIS, LEFR, MEGR, BLAN, DOBS, MOJ; l'anticorps monoclonal 5C5 ne se lie pas à un antigène de cellules de la lignée de carcinome rénal humain LAWR et ne se lie pas à l'antigène G250.

La grande spécificité de réaction de l'anticorps 5C5 a été démontrée par l'absence de réactivité de l'anticorps sur des cellules de lignées tumorales humaines autres que les lignées cellulaires de RCC. Ainsi l'anticorps monoclonal 5C5 ne se lie pas à un antigène de cellules de lignées de tumeurs du colon humaines: HRT18, HTL8R, LS174T, SW48, SW620 , ne se lie pas à un antigène de cellules de la lignée de tumeur du poumon humaine: LX2 , ne se lie pas à un antigène de cellules de la lignée de tumeur de l'estomac humaine: Kato , ne se lie pas à un antigène de cellules de lignées de tumeurs du sein humaines: McF7 et ZR75 , ne se lie pas à un antigène de cellules de la lignée de tumeur de l'ovaire humaine : OVCAR. Concernant la réactivité de l'anticorps sur les cellules normales, des expériences d'immunohistochimie ont révélé un marquage de l'anticorps monoclonal 5C5 dans le cytoplasme des tubes rénaux proximaux ainsi que le pôle apical et les faces latérales des cellules épithéliales coliques provenant de fragments tissulaires humains normaux.

La présente invention concerne également l'anticorps simple chaîne produit à partir de l'anticorps monoclonal selon l'invention, l'anticorps chimérique produit à partir de l'anticorps monoclonal selon l'invention et également l'anticorps humanisé produit à partir de l'anticorps monoclonal selon l'invention.

L'anticorps ou le fragment d'anticorps dirigé contre l'antigène de carcinome des cellules rénales humaines peut être administré pour provoquer une réponse immune anti-idiotypique ou anti-anti-idiotypique. Un anticorps anti-idiotypique dirigé contre l'anticorps réagissant contre un antigène de carcinome de cellules rénales humaines peut en effet exprimer des épitopes similaires à ceux de l'antigène et peut ainsi être utilisé de manière similaire pour l'immunisation et ainsi pour développer une réponse immune anti-cancéreuse. Un anticorps anti-anti-idiotypique peut être utilisé dans des voies similaires à celles décrites pour l'anticorps primaire anti-tumeur de la présente invention. En conséquence, il est également dans l'étendue de l'invention d'utiliser un anticorps anti-idiotypique caractérisé en ce qu'il est dirigé contre l'anticorps selon l'invention et d'utiliser un anticorps anti-anti-idiotypique caractérisé en ce qu'il est dirigé contre l'anticorps anti-idiotypique précédent.

L'invention concerne également un fragment de l'anticorps selon l'invention caractérisé en ce qu'il peut être utilisé à la place de l'anticorps entier. Dans un mode préféré de réalisation, ce fragment d'anticorps est un fragment F(ab')2, un fragment Fab' ou un fragment Fv.

Un autre mode de réalisation de l'invention concerne un agent de diagnostic de carcinome des cellules rénales humaines caractérisé en ce qu'il comprend un anticorps ou un fragment d'anticorps selon l'invention tel que décrit précédemment. Selon un mode préféré de réalisation, l'agent de diagnostic se caractérise en ce que l'anticorps ou le fragment d'anticorps est marqué directement ou indirectement par un marqueur générateur de signal; ce marqueur est sélectionné parmi les isotopes radioactifs et les entités non isotopiques; Les entités non isotopiques sont sélectionnées parmi les enzymes, les colorants, les haptènes, les agents luminescents tels que les agents radioluminescents, chémiluminescents, bioluminescents, fluorescents, phosphorescents, les ligands tels que la biotine, l'avidine, la streptavidine, la digoxygénine.

Selon un mode de réalisation, l'agent de diagnostic selon l'invention se caractérise en ce que l'anticorps ou le fragment d'anticorps est couplé à un support solide directement ou indirectement par l'intermédiaire d'un bras d'espacement. L'agent de diagnostic de l'invention peut être utilisé dans une méthode de diagnostic des RCC humains *in vitro* qui comprend la mise en contact d'un échantillon de fluide corporel et/ou de tissu corporel provenant d'un patient chez lequel on suspecte la présence d'un cancer avec un agent de diagnostic tel que précédemment décrit et la détermination de la présence d'un antigène ou d'un anticorps anti-idiotypique ou d'un anticorps selon l'invention. L'invention concerne également un kit de diagnostic caractérisé en ce qu'il contient l'agent de diagnostic décrit précédemment.

L'invention concerne également une composition pour la détection, la localisation et l'imagerie de carcinome de cellules rénales, comprenant un anticorps ou un fragment d'anticorps selon l'invention; les avantages de l'utilisation de fragments d'anticorps F(ab) ou F(ab)2' pour la détection, la localisation et l'imagerie ont été décrits par Goldenberg D.M. (Brevet US 4 331 647). L'anticorps ou le fragment d'anticorps selon l'invention est marqué directement ou indirectement avec un marqueur générateur de signal sélectionné parmi les isotopes radioactifs et les entités non isotopiques. Les radioisotopes émetteurs de rayons X, de rayons gamma, de positron et de fluorescence sont les plus appropriés pour le marquage des agents de diagnostic selon l'invention; comme radioisotopes utilisables pour le marquage de l'agent de diagnostic, on peut donc citer: Iode¹²³, Iode¹²⁵, Iode¹²⁶, Iode¹³¹, Iode¹³³, Brome⁷⁷, Technetium^{99m}, Indium¹¹¹, Indium^{113m}, Gallium⁶⁷, Gallium⁶⁸, Ruthénium⁹⁵, Ruthénium⁹⁷, Ruthénium¹⁰³, Ruthénium¹⁰⁵, Mercure¹⁰⁷, Mercure²⁰³, Rhénium^{99m}, Rhénium¹⁰¹, Rhénium¹⁰⁵, Scandium⁴⁷, Tellurium^{121m}, Tellurium^{122m}, Tellurium^{125m}, Thulium¹⁶⁵, Thulium¹⁶⁷, Thulium¹⁶⁸, Cuivre⁶⁷, Fluor¹⁸, Yttrium⁹⁰, Yttrium¹⁹⁹, Palladium¹⁰⁰, Bismuth²¹⁷, Antimoine²¹¹. Une variété de méthodes connues de l'homme de l'art existe pour accrocher des radioisotopes aux protéines soit directement soit via un agent chélatant tel le DTPA (Acide diéthylène triamine pentaacétique). Toutes ces méthodes peuvent être utilisées pour marquer les anticorps ou fragments d'anticorps de la présente invention. Par exemple l'anticorps monoclonal peut être marqué avec du Na[I¹²⁵] par la méthode à la chloramine T. [Hunter W.M. et Greenwood F.C. (1962) Nature 194: 495]. L'anticorps ou le fragment d'anticorps peut être directement marqué par du Technetium^{99m} par la technique de Crockford *et coll.* (brevet US 4 424 200) ou attaché via du DTPA comme décrit par Hnatowich (brevet US 4 479 930). Les entités non isotopiques sont sélectionnées parmi les enzymes, les colorants, les haptènes, les agents luminescents tels que les agents radioluminescents, chémiluminescents, bioluminescents, fluorescents, phosphorescents, les ligands tels que la biotine, l'avidine, la streptavidine, la digoxygénine.

L'invention concerne également une méthode de détection, de localisation et d'imagerie de carcinome de cellules rénales, comprenant les étapes suivantes (i) d'injection parentérale chez un être humain d'un anticorps ou d'un fragment d'anticorps selon l'invention, ou d'une composition telle que décrite ci-dessus; (ii) d'accumulation après un temps suffisant au niveau du site du carcinome de cellules rénales de l'anticorps marqué ou du fragment d'anticorps marqué sans que l'anticorps ou le fragment d'anticorps ne se lie de manière substantielle aux cellules normales; et (iii) de détection du signal au moyen d'un détecteur de signal puis (iv) de conversion du signal détecté en une image du carcinome de cellules rénales. Dans le cadre d'un marquage avec un radioisotope, le détecteur de signal utilisé est de préférence un équipement conventionnel tel une caméra gamma.

L'invention porte également sur une composition pharmaceutique destinée au traitement du carcinome rénal humain qui comprend une quantité thérapeutiquement efficace d'un anticorps ou d'un fragment d'anticorps selon l'invention et un véhicule pharmaceutiquement acceptable. Le véhicule pharmaceutiquement acceptable peut être tout type de véhicule employé habituellement dans la préparation de compositions injectables, c'est-à-dire un diluant, un agent de suspension tel une solution saline isotonique ou tamponnée. L'anticorps et/ou fragment d'anticorps selon l'invention peuvent être utilisés à titre de médicament; selon un mode de réalisation préféré, l'anticorps et/ou fragment d'anticorps selon l'invention sont utilisés à titre de médicament pour le traitement du carcinome de cellules rénales humaines; pour ce faire, l'anticorps ou le fragment d'anticorps de la composition pharmaceutique sont conjugués à au moins un agent sélectionné parmi le groupe des agents antiprolifératifs, antinéoplastiques ou cytotoxiques. Ces agents sont des radioisotopes ou des entités non isotopiques.

Dans un mode préféré de réalisation, l'agent antiprolifératif et/ou antinéoplastique et/ou cytotoxique non isotopique est une molécule d'acide nucléique. Cette molécule d'acide nucléique peut être de l'ADN simple brin, de l'ADN double brin, de l'ARN simple brin, de l'ARN double brin, un hybride ARN/ADN. Selon un mode préféré de réalisation, la molécule d'acide nucléique est de l'ADN double brin ou de l'ARN simple brin qui code pour un produit protéique d'intérêt qui s'exprime efficacement dans la cellule. Les produits protéiques d'intérêt sont choisis dans un groupe composé des interleukines, des cytokines, des lymphokines, des chémokines, des facteurs de croissance, des protéines tueuses, des protéines qui permettent de lever la chimiorésistance et des enzymes de restriction ; les interleukines, cytokines, lymphokines et chémokines sont choisies dans un groupe composé de préférence des interleukines I1-1, I1-2, I1-3, I1-4, I1-5, I1-6, I1-7, I1-8, I1-9, I1-10, I1-11, I1-12, I1-13, I1- 14, I1-15, I1-16, I1-17 et I1-18, des interférons α-IFN, β-IFN et γ-IFN ; de préférence le produit protéique d'intérêt est l'interleukine 2.

Selon un autre mode de réalisation de l'invention, la molécule d'acide nucléique est un ARN antisens.

La conjugaison de l'anticorps ou du fragment d'anticorps de la présente invention à un agent antiprolifératif, antinéoplastique ou cytotoxique tel que décrit ci-dessus peut être utilisé en thérapie génique. Selon un autre mode de réalisation, la conjugaison de l'anticorps ou du fragment d'anticorps de la présente invention à un agent antiprolifératif, antinéoplastique et cytotoxique peut être utilisé pour arrêter le développement des RCC et pour induire une régression et/ou une élimination de la masse tumorale. De préférence, l'anticorps ou le fragment d'anticorps ainsi conjugué est introduit dans le patient atteint de RCC et délivré aux sites tumoraux par voie orale ou parentérale dans un liquide transporteur pharmaceutiquement acceptable tel qu'une solution de sel physiologique. Alternativement, une solution ou une suspension d'anticorps ou de fragment d'anticorps conjugué à un agent peut être perfusée directement dans le tissu épithélial malin, cette méthode étant utilisée de préférence dans le cas où le RCC n'est pas métastasé.

Les radioisotopes préférés conjugués aux anticorps monoclonaux employés pour la thérapie sont des radioisotopes émetteurs de rayons gamma et de préférence l'Iode¹³¹, l'Yttrium⁹⁰, l'Or¹⁹⁹, le Palladium¹⁰⁰, le Cuivre⁶⁷, le Bismuth²¹⁷ et l'Antimoine²¹¹. Les radioisotopes émetteurs de rayons beta et alpha peuvent également être utilisés pour la thérapie. Les entités non isotopiques conjuguées aux anticorps monoclonaux employés pour la thérapie sont multiples et variés; on peut citer: (i) les antimétabolites telles les agents anti-folate, le méthotrexate, (ii) les analogues des purines et des pyrimidines (mercaptopurine, fluorouracile, 5-azacytidine), (iii) les antibiotiques, (iv) les lectines (ricine, abrine) et (iv) les toxines bactériennes (toxine diphtérique).

L'anticorps ou fragment d'anticorps selon l'invention est également utilisé en tant qu'agent de ciblage. Il permet ainsi de cibler des cellules cytotoxiques telles les cellules T humaines, les monocytes ou les cellules NK sur le lieu de la tumeur métastasée ou non. Les cellules cytotoxiques peuvent être attachées à l'anticorps via le récepteur Fc situé à la surface de ces cellules ou via un anticorps intermédiaire présentant une double spécificité par exemple; de tels anticorps bispécifiques pour le ciblage des carcinomes de cellules rénales peuvent être produits en fusionnant une cellule immunitaire produisant l'anticorps de la présente invention ou l'hydridome de la présente invention avec une cellule produisant un anticorps dirigé contre la cellule cytotoxique à cibler. Des anticorps bispécifiques peuvent également être produits par couplage chimique de deux anticorps ayant la spécificité désirée. L'anticorps ou fragment d'anticorps selon l'invention permet également de cibler des véhicules de délivrance d'agents antiprolifératifs, antinéoplastiques ou cytotoxiques sur le lieu de la tumeur métastasée ou non. Par véhicules de délivrance on entend désigner les liposomes et les particules virales.

Enfin, selon un autre mode préféré de réalisation, l'anticorps anti-idiotypique selon l'invention peut être utilisé pour la préparation d'un traitement prophylactique destiné au traitement du carcinome humain. En effet, l'anticorps anti-idiotype étant spécifiquement reconnu par l'anticorps ou le fragment d'anticorps selon l'invention, ils peuvent être utilisés pour l'immunisation afin de provoquer une réponse immune anti-cancéreuse dans l'organisme.

### LEGENDE DES FIGURES

**Figure 1:** Typage des lignées cellulaires de RCC en immunofluorescence indirecte par cytométrie en flux. Figure 1A: typage des lignées PIUZ et LAWR; figure 1B: typage des lignées DOBS et GUIL.
**Figure 2:** Mise en évidence de l'internalisation de l'anticorps 5C5 dans une lignée RCC.
   Les cellules du carcinome rénal ROB ont été incubées avec 5C5 conjugué à la fluorescéine (FITC) (A, C, E) et de la transferrine conjuguée au Texas-red (TRIC) (B, D, F). A 4°C, 5C5 marque la surface cellulaire (A). Après 10 minutes (C, D) et 30 minutes (E, F) d'internalisation, 5C5 (C, E) et la transferrine (D, F) sont internalisés et sont partiellement codistribués dans la région périnucléaire où est localisé le compartiment de recyclage tardif de la transferrine.

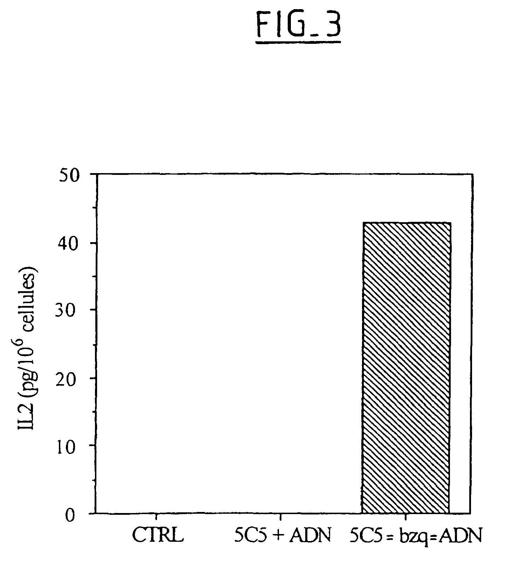

**Figure 3:** Production d'interleukine 2 (Il-2) de souris par des cellules ROB après transfection.

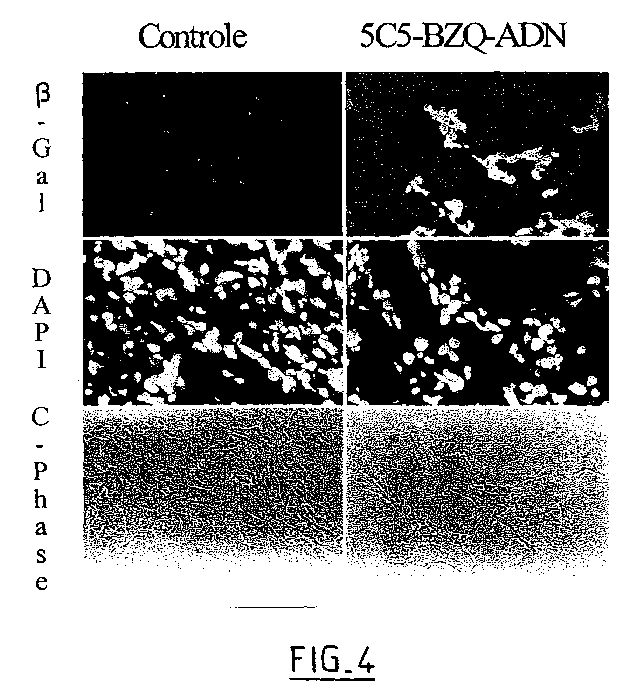

**Figure 4:** Expression de la β-galactosidase dans les RCC *in vivo*, après antifection intraveineuse d'un conjugué 5C5-benzoquinone-ADN.
   Une tumeur de carcinome rénal (HIEG) a été greffée sous la peau de souris Nude irradiées. Les souris ont secondairement reçu par voie intraveineuse de l'ADN complémentaire codant pour la β-galactosidase bactérienne, seul (Control) ou le même ADN conjugué à 5C5, via un couplage faisant intervenir la benzoquinone. La production de β-galactosidase a été mise en évidence par un marquage en immunofluorescence à l'aide d'un anticorps biotinylé dirigé contre la β-galactosidase (laboratoire Sigma), puis révélé à l'aide de la streptavidine FITC (b-Gal). Les noyaux des cellules de la tumeur sont visualisés à l'aide du DAPI et les cellules sont mises en évidence par contraste de phase (C-phase). La β-galactosidase n'est détectée dans les cellules tumorales que lorsque les souris ont reçu l'ADN conjugué à 5C5.

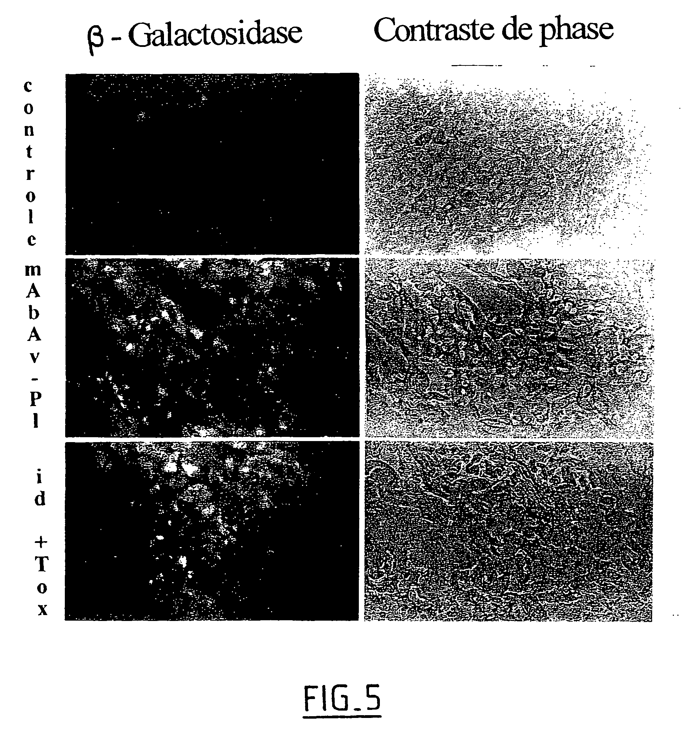

**Figure 5 :** Expression de la β-galactosidase dans les RCC *in vivo*, après antifection intraveineuse d'un conjugué 5C5-avidine-polyLysine-toxine-ADN.
   Une tumeur de carcinome rénal (HIEG) a été greffée sous la peau de souris Nude irradiées. Les souris ont secondairement reçu par voie intraveineuse de l'ADN complémentaire codant pour la β-galactosidase bactérienne, seul (control) ou le même ADN associé à un complexe avidine polylysine (via la benzoquinone) auquel est accroché 5C5 biotinylé (mAbAv-Pl) ou le même complexe comprenant en supplément la toxine bactérienne de hémagglutinine biotinylée permettant d'accroître l'efficacité du transfert de gène (id +Tox). La production de β-galactosidase a été déterminée par un marquage en immunofluorescence à l'aide d'un anticorps biotinylé dirigé contre la β galactosidase (laboratoire Sigma) puis révélé à l'aide de la streptavidine FITC (β-galactosidase). Les cellules sont mises en évidence par contraste de phase (Phase contrast). La β-galactosidase n'est détectée dans les cellules tumorales que lorsque les souris ont reçu l'ADN conjugué à 5C5.

D'autres caractéristiques et avantages de la présente invention apparaîtront à la lecture des exemples ci-après.

### EXEMPLES

### 1. Protocole de préparation de l'hybridome 5C5

Deux souris Balb/C ont reçu une injection intraplantaire de 4,5.10⁶ cellules du carcinome rénal humain BIZ fourni par le Dr Jean Gogusev (Hopital Necker-Enfants Malades, Paris, France) dans 100 µl de NaCl 0.9%.

Dix-huit jours plus tard, un rappel est réalisé avec 5.10⁶ cellules du carcinome rénal humain BIZ dans 50 µl de NaCl 0.9%. Trois jours plus tard, une suspension cellulaire est réalisée à partir des ganglions inguinaux et abdominaux des souris.

23.10⁶ cellules de la suspension cellulaire sont fusionnées avec 11,5.10⁶ cellules de myélome de souris Balb/C Sp2O, en présence de PEG4000. Le mélange cellulaire est ensuite déposé dans trois plaques de 96 puits remplies deux jours auparavant de cellules péritonéales de souris afin de ne sélectionner que les seules cellules hybrides capables de se développer.

Une première analyse par cytométrie en flux sur cellules BIZ a donné des résultats positifs pour le clone 5C5.

Une deuxième analyse a permis de confirmer ce résultat positif. Des ampoules 5C5 non clonées ont ainsi pu être congelées.

Une dilution limite à partir d'une ampoule décongelée du clone 5C5 a permis d'obtenir après analyse par cytométrie en flux sur cellules BIZ un sous clone 5C5. L'hybridome 5C5 a ainsi pu être congelé. La détermination de la classe et de la sous classe de l'anticorps monoclonal 5C5 a été réalisée à l'aide d'immunsérums de lapin spécifiques selon la technique d'Ouchterlony au Laboratoire d'Immunologie Expérimentale (collaboration Professeur H. BAZIN).

L'anticorps monoclonal 5C5 appartient à la classe des immunoglobulines IgG1k.

### 2. Caractérisation de l'anticorps monoclonal 5C5:

### 2.1. Sur les RCC

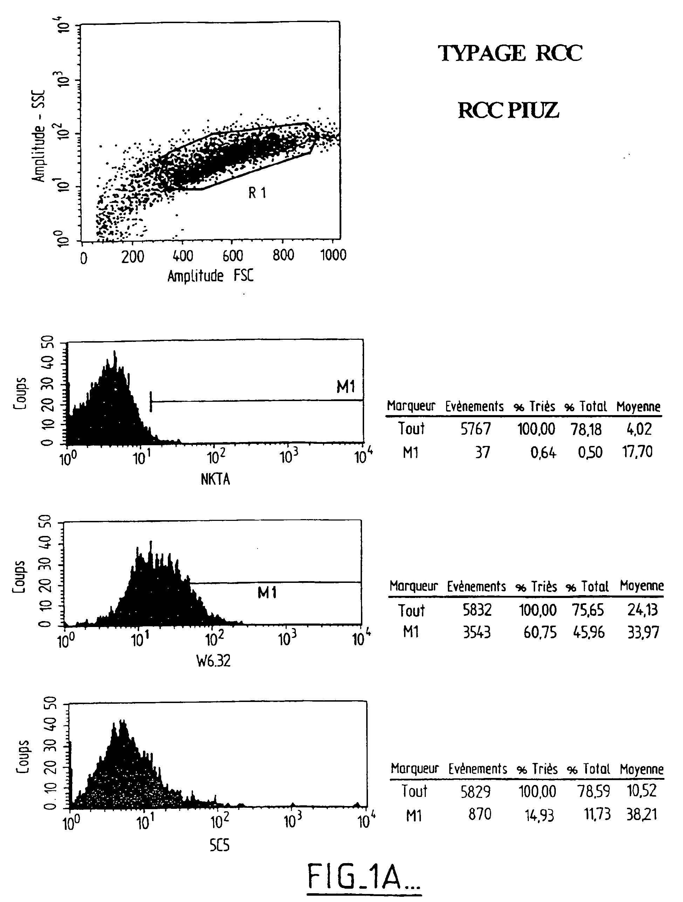

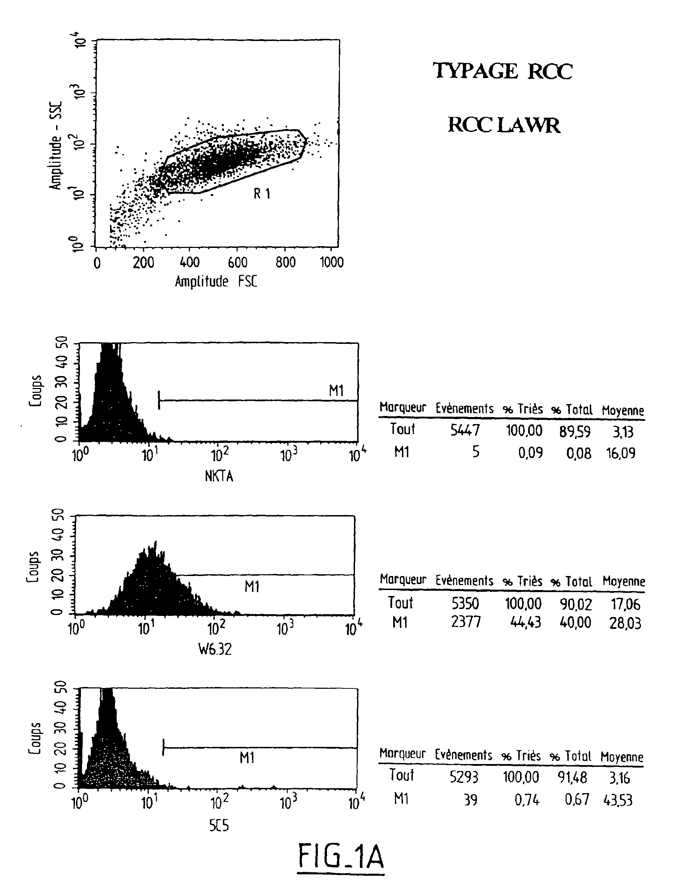

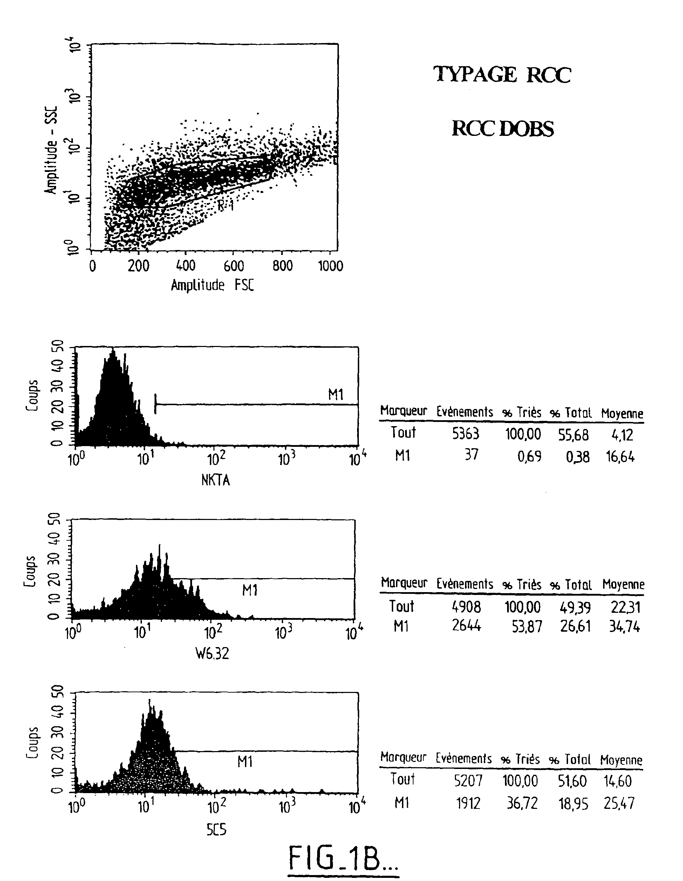

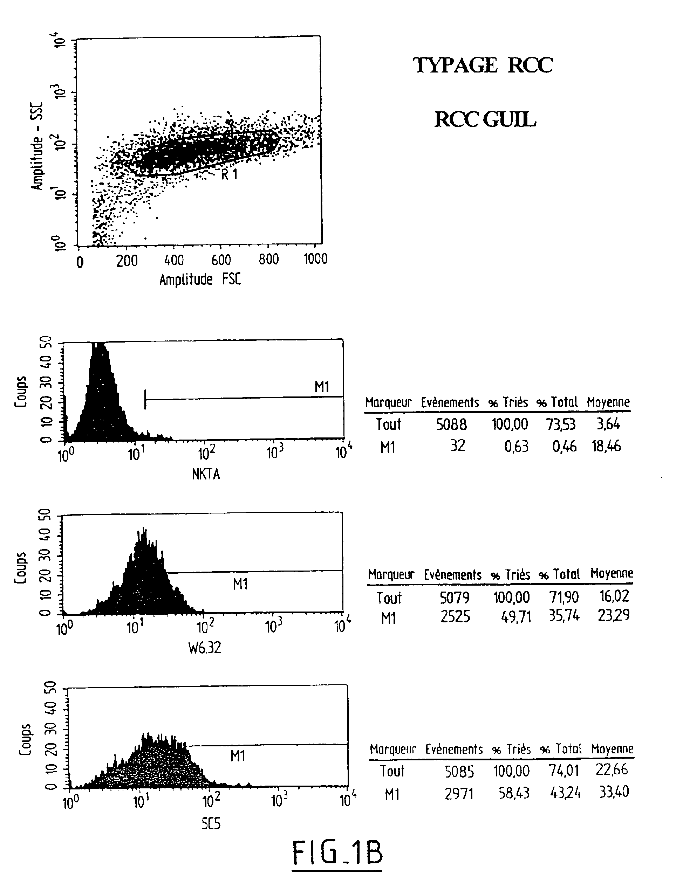

Dix-sept lignées de RCC humaines ont été testées en immunofluorescence indirecte, par cytométrie en flux, à l'aide du mAb 5C5, soit à partir de surnageants de culture, soit à partir de mAb immunopurifié sur colonne de protéine-A-Sepharose^{R}. Les 17 lignées RCC humaines testées sont les suivantes : BIZ, HIEG, QUIE, BENA, PIUZ, GUIL, GUER, ROB, DURI, JOUS, GUIS, LEFR, MEGR, BLAN, DOBS, MOJ, LAWR. Seule LAWR n'est pas reconnue par l'anticorps monoclonal 5C5 (Figure 1).

Une étude parallèle réalisée en présence de l'anticorps G250 sur l'ensemble de ces tumeurs a montré que les lignées BIZ et HIEG n'exprimaient pas l'anticorps G250 alors que les lignées ROB et QUIE l'expriment (résultats non montrés).

### 2.2. Sur d'autres lignées

L'anticorps monoclonal 5C5 a été testé sur diverses lignées tumorales humaines par immunofluorescence indirecte en microscopie (collaboration avec le Docteur J. LEPENDU, INSERM U 419, NANTES FRANCE). Les lignées testées sont les suivantes : HRT18, HLT8R, LS 174T, SW48 et SW620 dérivées de tumeur du colon, LX2 dérivée de tumeur du poumon, KATO dérivée du cancer de l'estomac, MCF7 et ZR75 dérivée du cancer du sein et OVCAR dérivée d'une tumeur de l'ovaire. Toutes ces expériences d'immunofluorescence indirecte utilisant l'anticorps monoclonal 5C5 sur ces lignées tumorales humaines se sont révélées négatives (résultats non montrés).

### 3- Utilisation de l'anticorps monoclonal 5C5 en thérapie génique in vitro:

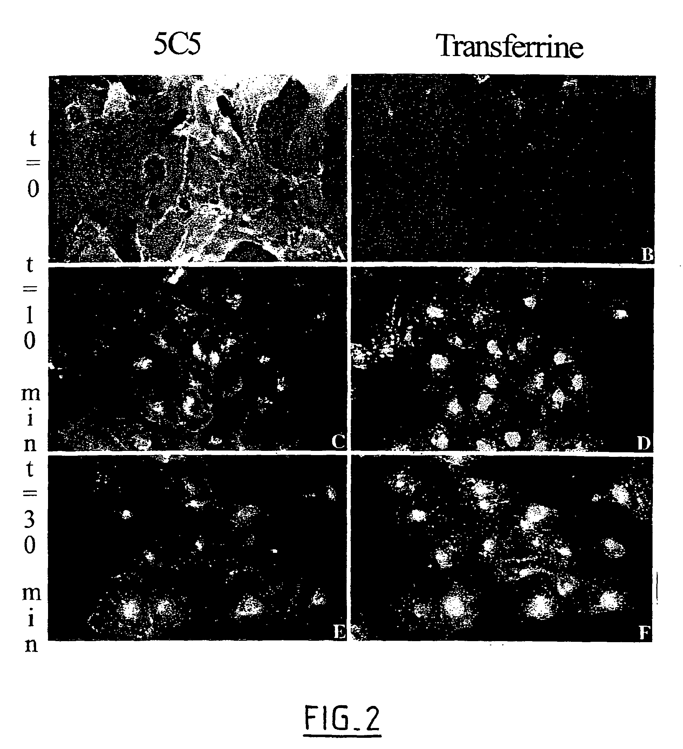

Les expériences d'immunofluorescence ont démontré que l'anticorps monoclonal 5C5 s'intemalise parfaitement dans les RCC (Figure 2). Ce résultat positif constitue un préalable indispensable à une approche vectorielle telle que l'antifection [Poncet *et al*. (1996) Gene Ther. 3: 731-738].

Les expériences d'antifection ont permis de démontrer que l'anticorps monoclonal 5C5 est apte à véhiculer un gène codant pour de l'interleukine-2 de souris (Il-2) dans les cellules; le gène de l'Il-2 murin ainsi véhiculé est transcrit et exprimé comme en témoigne la présence d'Il-2 dans les surnageant de culture (Figure 3).

### 4- Utilisation de l'AcM 5C5 en thérapie génique in vivo

Ces expériences ont été réalisées en collaboration avec E. Angevin (I.G.R.).

### 4. 1. 1 ^{ère} expérience

Des souris Nude préalablement irradiées ont reçu une greffe de tumeur HIEG en position sous-cutanée. Lorsque les tumeurs atteignent une taille d'environ 10x10 mm, les souris sont réparties en trois groupes :
* 1 souris naïve
* 2 souris ont reçu par voie intraveineuse rétro-orbitale, une injection d'un mélange de 10 µg de mAb 5C5 couplé à 100 µg de plasmide codant pour la β-galactosidase par l'intermédiaire de la benzoquinone, selon la technique décrite précédemment [Poncet *et al.* (1996) Gene Ther. 3:731-738].

Alors qu'aucune activité enzymatique n'a été retrouvée dans la tumeur de la souris naïve, par analyse histochimique, 2 souris sur les 3 ayant reçu le conjugué présentent une positivité marquée (Figure 4). Une étude plus exhaustive portant sur la recherche d'activité enzymatique a démontré pour les 2 souris positives son absence dans les autres organes testés et une positivité importante dans le foie de la souris négative.

### 4.2. 2^{ème} expérience

Des souris Nude préalablement irradiées ont reçu une greffe de tumeur HIEG en position sous-cutanée. L'anticorps monoclonal 5C5 préalablement biotinylé est couplé à un complexe incluant plusieurs composants, ADN=poly-L-lysines=benzoquinone=biotine via une molécule d'avidine. Enfin, une molécule d'exotoxine A de *Pseudomonas aeruginosa* biotinylée est rajoutée. Une activité rapporteur est recherchée par la mesure de l'expression de la β-galactosidase dans les tumeurs. Une forte activité β-galactosidase a été mise en évidence dans le groupe de souris ayant reçu le conjugué complet (Figure 5).

### REFERENCES

Holthafer *et al*. (1983) Lab. Invest. 49, 319-326.
Hunter W.M. et Greenwood F.C. (1962) Nature 194: 495.
Kaufmann O. *et al.* (1997) Histopathology 31:31-37.
Kinouchi T. *et al.* (1995) J. Urol. 154: 288-292.
Kôhler et Milstein (Nature (1975) 256: 495-497.
Oberling *et al*. (1960) Nature 186, 402-403.
Oosterwijk E. *et al.* (1993) J. Clin. Oncol. 11: 738-750.
Oosterwijk E *et al.* (1986) Int. J. Cancer 38: 489-494.
Poncet *et al.* (1996) Gene Therapy 3: 731-738.
Steffens M.G. *et al.* (1997) J. Clin. Oncol. 15: 1529-1537.

## Revendications

1. Hybridome désigné 5C5 tel que déposé à la CNCM sous le N° I-2184, formé par la fusion de cellules d'une lignée de myélome murin Balb/C Sp20 et de cellules de ganglions inguinaux et abdominaux de souris Balb/C ayant été préalablement immunisées avec la lignée de carcinome rénal humain BIZ.

2. Anticorps monoclonal 5C5 spécifique de la plupart des cellules de carcinomes rénaux humains, ne se liant pas à l'antigène G250 et susceptible d'être obtenu par l'hybridome de la revendication 1.

3. Anticorps simple chaîne produit à partir de l'anticorps monoclonal selon la revendication 2.

4. Anticorps chimérique produit à partir de l'anticorps selon la revendication 2 ou 3.

5. Anticorps humanisé produit à partir de l'anticorps selon la revendication 2 ou 3.

6. Anticorps anti-idiotypique **caractérisé en ce qu'**il est dirigé contre l'anticorps selon l'une quelconque des revendications 2 à 5.

7. Anticorps anti-anti-idiotypique **caractérisé en ce qu'**il est dirigé contre l'anticorps anti-idiotypique selon la revendication 6.

8. Fragment d'un anticorps selon l'une quelconque des revendications 2 à 7 **caractérisé en ce qu'**il conserve la spécificité dudit anticorps.

9. Fragment selon la revendication 8 **caractérisé en ce qu'**il s'agit d'un fragment F (ab') 2, d'un fragment Fab', ou d'un fragment Fv.

10. Agent de diagnostic de carcinome des cellules rénales humaines **caractérisé en ce qu'**il comprend un anticorps selon l'une des revendications 2 à 7 ou un fragment d'un anticorps selon les revendications 8 et 9.

11. Agent de diagnostic selon la revendication 10 **caractérisé en ce que** l'anticorps ou le fragment d'anticorps est marqué directement ou indirectement par un marqueur générateur de signal.

12. Agent de diagnostic selon la revendication 11 **caractérisé en ce que** le marqueur est sélectionné parmi les isotopes radioactifs et les entités non isotopiques.

13. Agent de diagnostic selon la revendication 12 **caractérisé en ce que** les entités non isotopiques sont sélectionnées parmi les enzymes, les colorants, les haptènes, les agents luminescents tels que les agents radioluminescents, chémiluminescents, bioluminescents, fluorescents, phosphorescents, les ligands tels que la biotine, l'avidine, la streptavidine, la digoxygénine.

14. Agent de diagnostic selon la revendication 10 **caractérisé en ce que** l'anticorps ou le fragment d'anticorps est couplé à un support solide directement ou indirectement par l'intermédiaire d'un bras d'espacement.

15. Méthode de diagnostic in vitro du carcinome rénal humain,comprenant :
- (i) la mise en contact d'un échantillon de fluide corporel et/ou de tissu corporel provenant d'un patient chez lequel on suspecte la présence d'un cancer avec un agent de diagnostic selon l'une quelconque des revendications 10 à 14 ; et
- (ii) la détermination de la présence d'un antigène de cellules de carcinome rénal humain susceptible d'être reconnu par un anticorps selon l'une quelconque des revendications 2 à 5 ou 7, ou par un de ses fragments selon les revendications 8 et 9 ou d'un anticorps anti-idiotypique selon la revendication 6, ou de la présence d'un anticorps selon les revendications 2 à 5 ou 7.

16. Kit de diagnostic **caractérisé en ce qu'**il contient un agent de diagnostic selon l'une quelconque des revendications 10 à 14.

17. Composition pour la détection, la localisation et l'imagerie de carcinome de cellules rénales, comprenant un anticorps selon l'une quelconque des revendications 2 à 5 ou 7, ou un fragment d'anticorps selon les revendications 8 ou 9, tel que l'anticorps ou le fragment d'anticorps est marqué directement ou indirectement avec un marqueur générateur de signal.

18. Composition selon la revendication 17 **caractérisée en ce que** le marqueur générateur de signal est sélectionné parmi les isotopes radioactifs et les entités non isotopiques.

19. Composition selon la revendication 18 **caractérisée en ce que** les isotopes radioactifs sont sélectionnés notamment parmi le groupe composé de l'Iode¹²³, l'Iode¹²⁵, l'Iode¹³¹, l'Indium¹¹¹, le Ruthénium⁹⁷, le Cuivre⁶⁷ ou le Technetium^{99m} et **en ce que** les entités non isotopiques sont sélectionnées parmi les enzymes, les colorants, les haptènes, les agents luminescents tels que les agents radioluminescents, chémiluminescents, bioluminescents, fluorescents, phosphorescents, les ligands tels que la biotine, l'avidine, la streptavidine, la digoxygénine.

20. Utilisation d'un anticorps selon l'une quelconque des revendications 2 à 5 ou 7 ou d'un fragment d'anticorps selon l'une quelconque des revendications 8 ou 9 ou d'une composition selon l'une des revendications 17 à 19 pour la préparation d'une composition destinée à la détection, à la localisation et à l'imagerie de carcinome de cellules rénales.

21. Composition pharmaceutique destinée au traitement du carcinome rénal humain qui comprend une quantité thérapeutiquement efficace d'un anticorps selon l'une quelconque des revendications de 2 à 7 ou d'un fragment d'anticorps selon l'une quelconque des revendications 8 à 9 et un véhicule pharmaceutiquement acceptable.

22. Anticorps selon l'une quelconque des revendications 2 à 7 et/ou fragment d'anticorps selon l'une des revendications 8 et 9 à titre de médicament.

23. Anticorps selon l'une quelconque des revendications 2 à 7 et/ou fragment d'anticorps selon l'une des revendications 8 et 9 à titre de médicament pour le traitement du carcinome de cellules rénales humaines.

24. Composition pharmaceutique selon la revendication 21 **caractérisée en ce que** ledit anticorps ou fragment d'anticorps est conjugué à au moins un agent sélectionné parmi le groupe des agents antiprolifératifs, antinéoplastiques ou cytotoxiques.

25. Composition pharmaceutique selon la revendication 24 **caractérisée en ce que** ledit agent est un isotope radioactif choisi parmi le groupe : l'Iode¹³¹, l'Yttrium⁹⁰, l'Or¹⁹⁹, le Palladium¹⁰⁰, le Cuivre⁶⁷, le Bismuth²¹⁷ et l'Antimoine²¹¹.

26. Anticorps selon l'une quelconque des revendications 2 à 5 ou 7, ou fragment d'anticorps selon la revendication 8 ou 9, en tant qu'agent de ciblage.

27. Utilisation d'un anticorps anti-idiotypique selon la revendication 6 pour la préparation d'une composition prophylactique destinée au traitement du carcinome humain.

## Claims

1. Hybridoma denoted 5C5, as deposited at the CNCM under number 1-2184, formed by fusing cells of a Balb/C murine myeloma line Sp20 and cells from inguinal and abdominal lymph nodes of Balb/C mice having been immunized beforehand with the BIZ human renal carcinoma line.

2. 5C5 Monoclonal antibody specific for most human renal carcinoma cells, which does not bind to the G250 antigen and which can be obtained with the hybridoma of Claim 1.

3. Single-chain antibody produced from the monoclonal antibody according to Claim 2.

4. Chimeric antibody produced from the antibody according to Claim 2 or 3.

5. Humanized antibody produced from the antibody according to Claim 2 or 3.

6. Anti-idiotypic antibody, **characterized in that** it is directed against the antibody according to any one of Claims 2 to 5.

7. Anti-anti-idiotypic antibody, **characterized in that** it is directed against the anti-idiotypic antibody according to Claim 6.

8. Fragment of an antibody according to any one of Claims 2 to 7, **characterized in that** it conserves the specificity of said antibody.

9. Fragment according to Claim 8, **characterized in that** it is an F(ab')2 fragment, an Fab' fragment or an Fv fragment.

10. Agent for diagnosing human renal cell carcinoma, **characterized in that** it comprises an antibody according to one of Claims 2 to 7 or an antibody fragment according to Claims 8 and 9.

11. Diagnostic agent according to claim 10, **characterized in that** the antibody or the antibody fragment is labeled directly or indirectly with a signal-generating label.

12. Diagnostic agent according to Claim 11, **characterized in that** the label is selected from radioactive isotopes and nonisotopic entities.

13. Diagnostic agent according to Claim 12, **characterized in that** the nonisotopic entities are selected from enzymes, dyes, haptens, luminescent agents such as radioluminescent, chemiluminescent, bioluminescent, fluorescent or phosphorescent agents, and ligands such as biotin, avidin, streptavidin or digoxigenin.

14. Diagnostic agent according to Claim 10, **characterized in that** the antibody or the antibody fragment is coupled to a solid support directly or indirectly via a spacer arm.

15. Method for diagnosing human renal carcinoma in vitro, comprising:
- (i) bringing a sample of body fluid and/or of body tissue originating from a patient in whom the presence of a cancer is suspected into contact with a diagnostic agent according to any one of Claims 10 to 14; and
- (ii) determining the presence of a human renal carcinoma cell antigen which can be recognized by an antibody according to any one of Claims 2 to 5 or 7, or by one of its fragments according to Claims 8 and 9 or of an anti-idiotypic antibody according to Claim 6, or the presence of an antibody according to Claims 2 to 5 or 7.

16. Diagnostic kit, **characterized in that** it contains a diagnostic agent according to any one of Claims 10 to 14.

17. Composition for detecting, localizing and imaging renal cell carcinoma, comprising an antibody according to any one of Claims 2 to 5 or 7, or an antibody fragment according to claim 8 or 9, such that the antibody or the antibody fragment is labeled directly or indirectly with a signal-generating label.

18. Composition according to Claim 17, **characterized in that** the signal-generating label is selected from radioactive isotopes and nonisotopic entities.

19. Composition according to Claim 18, **characterized in that** the radioactive isotopes are selected, in particular, from the group composed of Iodine¹²³, Iodine¹²⁵, Iodine¹³¹, Indium¹¹¹, Ruthenium⁹⁷, Copper⁶⁷ and Technetium^{99m}, and **in that** the nonisotopic entities are selected from enzymes, dyes, haptens, luminescent agents such as radioluminescent, chemiluminescent, bioluminescent, fluorescent or phosphorescent agents, and ligands such as biotin, avidin, streptavidin or digoxigenin.

20. Use of an antibody according to any one of Claims 2 to 5 or 7 or of an antibody fragment according to any one of Claims 8 or 9 or of a composition according to one of Claims 17 to 19, for preparing a composition intended for detecting, localizing and imaging renal cell carcinoma.

21. Pharmaceutical composition intended to treat human renal carcinoma, which comprises a therapeutically effective amount of an antibody according to any one of Claims 2 to 7 or of an antibody fragment according to any one of Claims 8 to 9, and a pharmaceutically acceptable vehicle.

22. Antibody according to any one of Claims 2 to 7 and/or antibody fragment according to one of Claims 8 and 9, as a medicinal product.

23. Antibody according to any one of Claims 2 to 7 and/or antibody fragment according to one of Claims 8 and 9, as a medicinal product for treating human renal cell carcinoma.

24. Pharmaceutical composition according to Claim 21, **characterized in that** said antibody or antibody fragment is conjugated to at least one agent selected from the group of antiproliferative, antineoplastic or cytotoxic agents.

25. Pharmaceutical composition according to Claim 24, **characterized in that** said agent is a radioactive isotope chosen from the group: Iodine¹³¹, Yttrium⁹⁰, Gold¹⁹⁹, Palladium¹⁰⁰, Copper⁶⁷, Bismuth²¹⁷ and Antimony²¹¹.

26. Antibody according to any one of Claims 2 to 5 or 7 or antibody fragment according to Claim 8 or 9, as a targeting agent.

27. Use of an anti-idiotypic antibody according to Claim 6, for preparing a prophylactic composition intended to treat human carcinoma.

## Patentansprüche

1. Als 5C5 bezeichnetes Hybridom, wie es bei der CNCM unter der Nr. 1-2184 hinterlegt worden ist, welches durch die Fusion von Zellen einer Balb/C Sp20-Mäuse-Myelomlinie und von Zellen von inguinalen und abdominalen Ganglions von Balb/C-Mäusen, die vorab mit der humanen Nierenkarzinomlinie BIZ immunisiert worden sind, gebildet wird.

2. Monoklonaler 5C5-Antikörper, der für den Hauptteil der Zellen von humanen Nierenkarzinomen spezifisch ist, der sich nicht an das Antigen G250 bindet und durch das Hybridom des Anspruchs 1 erhalten werden kann.

3. Einzelkettiger Antikörper, welcher ausgehend von dem monoklonalen Antikörper nach Anspruch 2 hergestellt wird.

4. Chimärer Antikörper, der ausgehend von dem Antikörper nach Anspruch 2 oder 3 hergestellt wird.

5. Humanisierter Antikörper, der ausgehend von dem Antikörper nach Anspruch 2 oder 3 hergestellt wird.

6. Anti-idiotypischer Antikörper, **dadurch gekennzeichnet, dass** er gegen den Antikörper nach einem der Ansprüche 2 bis 5 gerichtet ist.

7. Anti-anti-idiotypischer Antikörper, **dadurch gekennzeichnet, dass** er gegen den anti-idiotypischen Antikörper nach Anspruch 6 gerichtet ist.

8. Fragment eines Antikörpers nach einem der Ansprüche 2 bis 7, **dadurch gekennzeichnet, dass** er die Spezifität des Antikörpers bewahrt.

9. Fragment nach Anspruch 8, **dadurch gekennzeichnet, dass** es sich um ein F(ab')2-Fragment, ein Fab'-Fragment oder ein Fv-Fragment handelt.

10. Mittel zur Diagnose eines Karzinoms von humanen Nierenzellen, **dadurch gekennzeichnet, dass** es einen Antikörper nach einem der Ansprüche 2 bis 7 oder ein Fragment eines Antikörpers nach den Ansprüche 8 und 9 umfasst.

11. Mittel zur Diagnose nach Anspruch 10, **dadurch gekennzeichnet, dass** der Antikörper oder das Antikörperfragment direkt oder indirekt durch einen Marker, welcher ein Signal erzeugt, markiert ist.

12. Mittel zur Diagnose nach Anspruch 11, **dadurch gekennzeichnet, dass** der Marker unter den radioaktiven Isotopen und den nicht-isotopischen Entitäten ausgewählt wird.

13. Mittel zur Diagnose nach Anspruch 12, **dadurch gekennzeichnet, dass** die nicht-isotopischen Entitäten unter den Enzymen, den Färbemitteln, den Haptenen, den lumineszierenden Mitteln, wie den radiolumineszierenden, chemolumineszierenden, biolumineszierenden, fluoreszierenden, phosphoreszierenden Mitteln, den Liganden, wie Biotin, Avidin, Streptavidin, Digoxygenin, ausgewählt werden.

14. Mittel zur Diagnose nach Anspruch 10, **dadurch gekennzeichnet, dass** der Antikörper oder das Antikörperfragment direkt oder indirekt durch das Zwischenglied eines Spacer-Arms an einen festen Träger gekoppelt ist.

15. In vitro-Verfahren zur Diagnose des humanen Nierenkarzinoms, umfassend:
- (i) das Inkontaktbringen einer Probe von Körperfluid und/oder von Körpergewebe, welches von einem Patienten stammt, bei welchem man das Vorliegen einer Krebserkrankung vermutet, mit einem Mittel zur Diagnose nach einem der Ansprüche 10 bis 14; und
- (ii) die Bestimmung des Vorhandenseins eines Antigens von Zellen eines humanen Nierenkarzinoms, welches durch einen Antikörper nach einem der Ansprüche 2 bis 5 oder 7 oder durch eines von dessen Fragmenten nach den Ansprüchen 8 und 9 erkannt werden kann, oder eines anti-idiotypischen Antikörpers nach Anspruch 6 oder des Vorhandenseins eines Antikörpers nach den Ansprüchen 2 bis 5 oder 7.

16. Diagnostischer Kit, **dadurch gekennzeichnet, dass** er ein Mittel zur Diagnose nach einem der Ansprüche 10 bis 14 enthält.

17. Zusammensetzung für den Nachweis, die Lokalisierung und die Bildgebung eines Karzinoms von Nierenzellen, umfassend einen Antikörper nach einem der Ansprüche 2 bis 5 oder 7 oder ein Antikörperfragment nach den Ansprüchen 8 oder 9 derart, dass der Antikörper oder das Antikörperfragment direkt oder indirekt mit einem Marker, welcher ein Signal erzeugt, markiert ist.

18. Zusammensetzung nach Anspruch 17, **dadurch gekennzeichnet, dass** der Marker, welcher ein Signal erzeugt, unter den radioaktiven Isotopen und den nicht-isotopischen Entitäten ausgewählt wird.

19. Zusammensetzung nach Anspruch 18, **dadurch gekennzeichnet, dass** die radioaktiven Isotope insbesondere aus der aus Iod¹²³, Iod¹²⁵, Iod¹³¹, Indium¹¹¹, Ruthenium⁹⁷, Kupfer⁶⁷ oder Technetium^{99m} bestehenden Gruppe ausgewählt werden und dass die nicht-isotopischen Entitäten unter den Enzymen, den Färbemitteln, den Haptenen, den lumineszierenden Mitteln, wie den radiolumineszierenden, chemolumineszierenden, biolumineszierenden, fluoreszierenden, phosphoreszierenden Mitteln, den Liganden, wie Biotin, Avidin, Streptavidin, Digoxigenin, ausgewählt werden.

20. Verwendung eines Antikörpers nach einem der Ansprüche 2 bis 5 oder 7 oder eines Antikörperfragments nach einem der Ansprüche 8 oder 9 oder einer Zusammensetzung nach einem der Ansprüche 17 bis 19 für die Herstellung einer Zusammensetzung, die für den Nachweis, die Lokalisierung und die Bildgebung eines Karzinoms von Nierenzellen bestimmt ist.

21. Pharmazeutische Zusammensetzung, welche für die Behandlung des humanen Nierenkarzinoms bestimmt ist, welche eine therapeutisch wirksame Menge eines Antikörpers nach einem der Ansprüche 2 bis 7 oder von einem Antikörperfragment nach einem der Ansprüche 8 bis 9 und einen pharmazeutisch annehmbaren Träger umfasst.

22. Antikörper nach einem der Ansprüche 2 bis 7 und/oder Antikörperfragment nach einem der Ansprüche 8 bis 9 als Arzneimittel.

23. Antikörper nach einem der Ansprüche 2 bis 7 und/oder Antikörperfragment nach einem der Ansprüche 8 bis 9 als Arzneimittel für die Behandlung des humanen Nierenzellkarzinoms.

24. Pharmazeutische Zusammensetzung nach Anspruch 21, **dadurch gekennzeichnet, dass** der Antikörper oder das Antikörperfragment mit wenigstens einem Mittel, welches aus der Gruppe der antiproliferativen, antineoplastischen oder zytotoxischen Mittel ausgewählt wird, konjugiert ist.

25. Pharmazeutische Zusammensetzung nach Anspruch 24, **dadurch gekennzeichnet, dass** das Mittel ein radioaktives Isotop ist, welches aus der Gruppe: Iod¹³¹, Yttrium⁹⁰, Gold¹⁹⁹, Palladium¹⁰⁰, Kupfer⁶⁷, Bismut²¹⁷ und Antimon²¹¹ ausgewählt wird.

26. Antikörper nach einem der Ansprüche 2 bis 5 oder 7 oder Antikörperfragment nach Anspruch 8 oder 9 als Targeting-Mittel.

27. Verwendung eines anti-idiotypischen Antikörpers nach Anspruch 6 für die Herstellung einer prophylaktischen Zusammensetzung, welche für die Behandlung von Karzinomen beim Menschen bestimmt ist.
